# EUROPEAN PATENT APPLICATION

(11) **EP 4 850 702 A1**
(43) Date of publication of application: **09.12.2026**
(21) Application number: 25382579.8
(22) Date of filing: 05.06.2025
(51) Int. Cl.: A61K 49/10, A61K 49/18

(54) **FLUORINE-19-CONTAINING NANOPARTICLES FOR USE IN A IN VIVO METHOD OF IMAGING THROMBI IN A SUBJECT, WHEREIN THE NANOPARTICLES SERVE AS CONTRAST AGENTS**

(71) Applicant: Fundación Pública Galega Instituto de Investigación Sanitaria de Santiago de Compostela, 15706 Santiago de Compostela A Coruña (ES)
(72) Inventor: HERVELLA LORENZO, Pablo, 15706 Santiago de Compostela (ES); IGLESIAS REY, Ramón, 15706 Santiago de Compostela (ES)
(74) Representative: Hoffmann Eitle

(57) **Abstract**

The present invention refers to fluorine-19-containing nanoparticles for use in a *in vivo* method of imaging thrombi in a subject, wherein the nanoparticles serve as contrast agents.

## Description

### FIELD OF THE INVENTION

The present invention refers to the medical field. Particularly, the present invention refers to fluorine-19-containing nanoparticles for use in a *in vivo* method of imaging thrombi in a subject, wherein the nanoparticles serve as contrast agents.

### STATE OF THE ART

Ischemic stroke is a major global health challenge with a profound clinical and socioeconomic impact. In Spain, it is the second leading cause of death and the most common cause of death among women. It is also the leading cause of acquired disability in adults and the second leading cause of dementia. Approximately 120,000 people experience a stroke annually in Spain, with half of them suffering from lasting disabilities that severely affect their independence, and a quarter dying. Stroke accounts for 70% of neurological admissions and approximately 6% of overall healthcare expenditure in Spain. This burden is expected to rise due to an aging population and the associated increase in stroke incidence.

The vast majority of strokes (around 80%) are ischemic in origin, caused by the thrombotic occlusion of a cerebral artery. Current treatment strategies aim to restore blood flow through the administration of fibrinolytic agents (e.g., tPA) or by means of endovascular procedures such as mechanical thrombectomy. However, these treatments have limitations: fibrinolytic therapy is restricted to a narrow time window and carries a risk of hemorrhagic transformation, while mechanical thrombectomy is often less effective in distal occlusions, which account for a substantial proportion of ischemic strokes.

It is now well established that the composition and structural characteristics of thrombi significantly influence treatment outcomes. Nevertheless, no clinically available imaging method currently provides direct, accurate, and specific information about thrombus characteristics *in vivo.* The absence of such molecular imaging tools hampers patient stratification and individualized therapeutic decision-making. As a result, clinicians must rely on indirect markers or general anatomical imaging, which do not offer sufficient insight into thrombus composition, structure, or biological behavior.

There is therefore an unmet clinical need for an imaging strategy that can provide real-time, *in vivo,* non-invasive information about thrombi, not only to localize them precisely but also to understand their biological and structural characteristics. Such a strategy could significantly enhance clinical decision-making, improve treatment outcomes, and reduce both morbidity and mortality. In addition, earlier and more precise treatment selection could lower healthcare costs and reduce the personal and social burden on caregivers, who are often women and who frequently experience discrimination due to the caregiving role.

The present invention is focused on solving this problem and an innovative strategy for imaging thrombi in a subject is herein provided.

### Description of the invention

### Brief description of the invention

The present invention refers to a contrast agent for molecular imaging, preferably by magnetic resonance imaging (MRI), specifically formulated for *in vivo* visualization, localization, and/or characterization of thrombi. The invention is particularly useful for identifying thrombi during the acute phase of cerebral ischemia, addressing an unmet clinical need for more precise and personalized diagnostic tools in stroke management.

The invention provides fluorine-19 (¹⁹F)-containing lipid-based nanoparticles that serve as targeted contrast agents, preferably in MRI. These nanoparticles are designed to selectively accumulate at thrombus sites, thereby enabling the detection of fluorine-19 signals associated with the thrombi. This allows clinicians to obtain real-time images with high specificity and minimal background interference, given the absence of endogenous fluorine-19 in human tissues.

The present invention involves administering the nanoparticles to a subject, acquiring MRI images from the relevant anatomical region, and analyzing the images to identify the presence, location, and characteristics of thrombi based on the detected fluorine-19 signal. The invention further enables differentiation between fresh, platelet-rich thrombi and older, fibrin-rich thrombi, which is clinically relevant for determining the most appropriate treatment (e.g., thrombolysis vs. thrombectomy).

The fluorine-19 nanoparticles of the invention exhibit several preferred structural features:
- A lipid-based matrix comprising preferably 1,2-Distearoyl-sn-glycero-3-phosphocholine (DSPC) and cholesterol, forming stable and biocompatible vesicles;
- Encapsulation of perfluorinated compounds such as, for example, perfluorooctyl bromide (PFOB), perfluorooctane, or perfluorodecalin, which provide a strong and specific MRI signal due to the favorable nuclear properties of fluorine-19;
- Functionalization with platelet plasma membranes, conferring thrombus-targeting capabilities by presenting biologically relevant surface markers involved in thrombus formation;
- Surface coating with polyethylene glycol (PEG), enhancing nanoparticle stability, circulation time, solubility, and overall biocompatibility.

This combination of targeted delivery, strong and specific MRI signal, and favourable safety profile, especially compared to traditional gadolinium-based agents, makes the invention a highly valuable diagnostic tool. It offers the potential to improve stroke diagnosis, guide clinical decision-making, reduce treatment delays, and ultimately improve patient outcomes.

So, the first embodiment of the present invention refers to fluorine-19-containing nanoparticles specifically designed for use as contrast agents in *in vivo* MRI imaging of thrombi in a subject. These nanoparticles are particularly suited for the visualization, localization, and/or characterization of thrombi in a subject, especially during the acute phase of cerebral ischemia.

In one embodiment, the invention provides a nanoparticle formulation comprising a lipid matrix encapsulating a fluorinated compound selected from perfluorooctyl bromide (PFOB), perfluorooctane, or perfluorodecalin. The lipid matrix is preferably composed of 1,2-Distearoyl-sn-glycero-3-phosphocholine (DSPC) and cholesterol, which can be mixed in a molar ratio of approximately 2:1 to form a stable and biocompatible structure. The resulting nanoparticles typically have a diameter ranging between 100 and 200 nanometers, which allows for optimal circulation in the bloodstream and efficient penetration into thrombotic regions.

In a preferred embodiment, these nanoparticles are further functionalized with platelet plasma membranes to confer thrombus-targeting capabilities. Platelet membrane coating provides the nanoparticles with surface proteins and antigens naturally involved in thrombus formation and adhesion, such as GPIIb/IIIa. This functionalization enhances the selective accumulation of the nanoparticles at thrombus sites, particularly those enriched in platelets. The platelet membrane can be derived from rat platelets and coated onto the nanoparticles through sonication followed by extrusion through 200 nm polycarbonate filters to ensure unilamellar membrane deposition and uniform size distribution.

In another embodiment, the nanoparticles are modified by coating with polyethylene glycol (PEG), preferably through the incorporation of PEGylated lipids such as DSPE-PEG2000 into the lipid matrix. This PEG coating improves nanoparticle stability in biological fluids, increases circulation half-life by reducing recognition by the reticuloendothelial system, and enhances solubility and biocompatibility. The PEG content is generally maintained within 5 to 10 mol% relative to the total lipid content to achieve an optimal balance between stealth properties and functional performance.

The invention also encompasses a method for imaging thrombi *in vivo* using the aforementioned nanoparticles. The method typically involves administering the nanoparticles to a subject, acquiring images of the relevant anatomical region, and analyzing the resulting images to visualize, localize, and characterize thrombi. MRI using fluorine-19 is the preferred modality due to its high sensitivity, excellent spatial resolution, and the absence of endogenous fluorine background in human tissues.

Importantly, the nanoparticles of the invention allow for the differentiation between fresh, platelet-rich thrombi and older, fibrin-rich thrombi based on their molecular composition and binding affinity to the targeted nanoparticle formulation. In experimental observations, platelet-rich thrombi demonstrate a higher and more rapid accumulation of nanoparticles, with peak signal intensity detected within the first 30 minutes post-injection. This feature enables clinicians to determine the age and composition of the thrombus, which is critical for selecting the most appropriate treatment strategy, such as pharmacological thrombolysis versus mechanical thrombectomy.

The invention further includes a method of preparing the nanoparticles. This process involves first preparing a lipid mixture, for example comprising DSPC and cholesterol, followed by encapsulation of the fluorinated compound, for example via nanoprecipitation or emulsification methods, such as ethanol injection under sonication. The resulting lipid-based nanoparticles are then functionalized with platelet plasma membranes through sonication and membrane extrusion. Optionally, a PEG coating is applied by incorporating PEGylated lipids into the formulation either during or after nanoparticle formation.

Overall, the invention provides a multifunctional contrast agent that not only enhances thrombus imaging but also provides molecular-level insight into thrombus composition. By enabling the use of multiple imaging modalities and allowing real-time distinction between different thrombus types, this technology addresses critical unmet needs in stroke diagnosis and management, facilitating faster and more precise therapeutic decision-making and ultimately improving patient outcomes.

For the purpose of the present invention the following terms are defined:
- "Comprising" means "including but not limited to." When a claim uses comprising, it allows for the presence of additional elements or steps beyond those explicitly mentioned in the claim. So, it includes the claimed elements, but other components may also be present.
- "Consisting of" is a closed-ended transitional phrase. It limits the scope of the claim to only the elements or steps specifically listed. No additional elements or steps can be included.
- "Contrast agents" are substances used in medical imaging to enhance the contrast of structures or fluids within the body. They improve the visibility of specific tissues, organs, or pathologies in imaging techniques such as MRI, CT, or ultrasound.
- "Imaging thrombi" refers to the process of visualizing blood clots (thrombi) within the body using medical imaging techniques. This may involve the use of contrast agents or targeted probes to enhance the detection and localization of thrombi in blood vessels or tissues, typically through modalities such as MRI, computed tomography (CT), positron emission tomography (PET), or ultrasound.

### Description of the figures

**Figure 1****.** Example of the structure of ¹⁹F-based components.
**Figure 2****.** Diagram of the 19F -NP with PFOB.
**Figure 3****.** A schematic illustration of a 19F-containing nanoparticle encapsulating PFOB and coated with a platelet membrane for functionalization.
**Figure 4****.** Analysis of particle size and polydispersity index (PDI) in relation to the phospholipid composition of the samples described in Table 2.
**Figure 5****.** Time-evolution analysis of particle size and polydispersity index (PDI) for the NPs described in Table 3.
**Figure 6****.** Time-evolution analysis of particle size and polydispersity index (PDI) comparing lipid shell optimization vs. PFOB core optimization in NPs.
**Figure 7****.** TEM image of the NPs described in Table 3.
**Figure 8****.** Example of MR spectra used to 19F quantification inside NPs.
**Figure 9****.** Representative MR images demonstrating signal intensity across varying concentrations of PFOB, accompanied by a graphical representation of signal-to-concentration correlation.
**Figure 10****.** Representative MR repetition time (TR) and echo time (TE) for T1 and T2 determination.
**Figure 11****.** *In vitro* stability: An agarose phantom was prepared by embedding a mixture of blood and ¹⁹F-labeled NPs within a sample holder, simulating a biologically relevant environment for MRI assessment. This setup allowed for controlled evaluation of signal behavior in the presence of blood components while maintaining a stable and reproducible structure for imaging. The results demonstrated that the signal intensity remained consistent, indicating high in vitro stability under physiological conditions.

### Detailed description of the invention

The present invention is further illustrated by the following Examples, which are provided for illustrative purposes only and are not intended to limit the scope of the invention.

### Example 1. Material and methods

### Example 1.1. Synthesis of Nanoparticles (NPs)

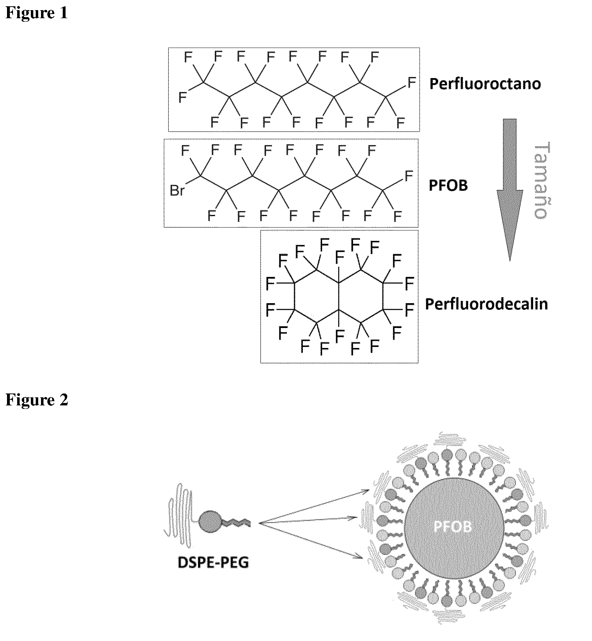

The NPs are synthesized using a nanoprecipitation technique, a widely employed method for producing stable and homogeneous nanocarriers. The nanoparticle matrix comprises distearoyl-sn-glycero-3-phosphocholine (DSPC), a phospholipid that imparts structural stability and biocompatibility, and cholesterol, which enhances membrane rigidity and preserves encapsulation integrity. These components serve as encapsulating agents for fluorinated compounds such as perfluorooctyl bromide (PFOB), perfluorooctane, or perfluorodecalin. Figure 1 illustrates representative 19F-containing compounds. Figure 2 provides a schematic representation of a 19F-containing nanoparticle encapsulating PFOB.

### Example 1.2. Functionalization of NPs

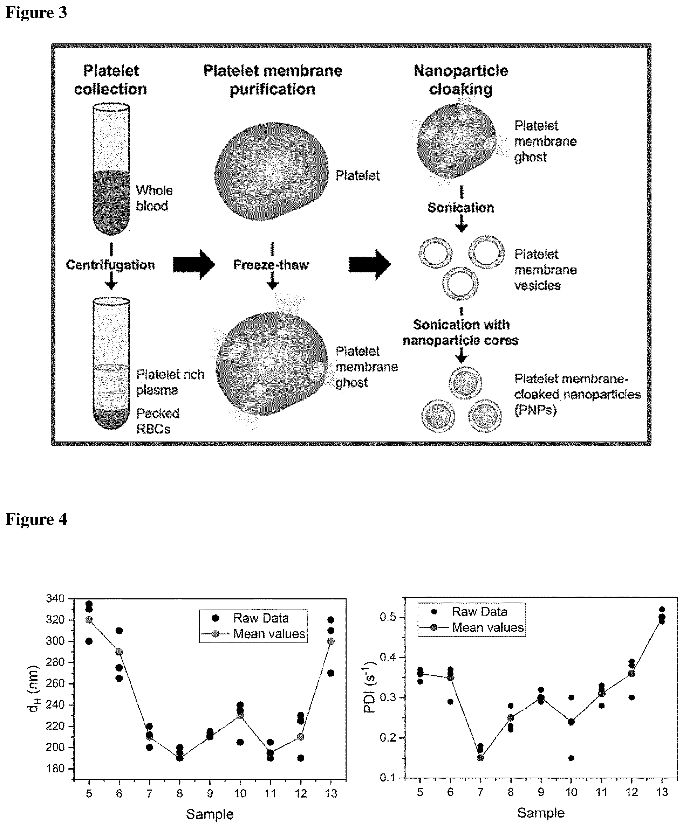

Polyethylene glycol (PEG) confers multiple advantages to NPs, including improved aqueous solubility, enhanced colloidal stability, and increased biocompatibility, thereby facilitating the application of 19F-NPs in *in vivo* settings. To enable thrombus-targeting capability, the NPs are further coated with rat platelet plasma membranes. This coating results in functionalized unilamellar membranes bearing platelet-derived antigens, which promote selective accumulation at sites of thrombus formation. **Figure 3** provides a schematic representation of a 19F-containing nanoparticle encapsulating PFOB and functionalized with a platelet membrane coating.

### Example 1.3. Characterization of NPs

The NPs are characterized in terms of their size and morphology using dynamic light scattering (DLS) and transmission electron microscopy (TEM), respectively. The presence and quantification of 19F-containing compounds encapsulated within the NPs are confirmed by nuclear magnetic resonance spectroscopy (NMR). MRI performance and signal quantification are evaluated using a 9.4 T Bruker BIOSPEC 94/20 system.
- *In vitro* studies: *In vitro* studies include assessment of stability, cytotoxicity and evaluation of thrombus-binding affinity of the functionalized 19F-NPs.
- *In vivo* imaging of cerebrovascular thrombi. The efficacy of the 19F-NPs for imaging cerebral thrombi is assessed in a thromboembolic stroke model, using animal models previously described in the literature.

### Example 2. Results

### Example 2.1. Dynamic Light Scattering (DLS)

The size, polydispersity index (PDI), and surface charge of the nanoparticles (NPs) were studied using DLS. It was demonstrated that surface functionalization with polyethylene glycol (PEG) must be performed after NP formation to ensure colloidal stability and prevent aggregation. Additionally, cholesterol presence in the phospholipid bilayer was confirmed as a key factor in maintaining NP stability over time.

**Table 1** summarizes the radius and PDI of NPs formulated with three different 19F compounds (perfluorooctyl bromide [PFOB], perfluorooctane, and perfluorodecalin) over 10 days. All three fluorinated agents were successfully encapsulated; however, PFOB was selected as the primary agent due to its superior biocompatibility, low toxicity, and efficient incorporation into the NP formulation.

**Table 1**

| **Parameter** | **Day 0** | **Day 2** | **Day 5** | **Day 10** |
|---|---|---|---|---|
| **PFOB radius (nm)** | 85.06 ± 0.45 | 90.10 ± 0.25 | 92.11 ± 0.31 | 89.11 ± 0.27 |
| **PFOB PDI** | 0.190 ± 0.011 | 0.170 ± 0.004 | 0.165 ± 0.012 | 0.182 ± 0.009 |
| **Perfluorooctane radius (nm)** | 103.11 ± 0.30 | 107.20 ± 0.35 | 106.41 ± 0.38 | 104.00 ± 0.39 |
| **Perfluorooctane PDI** | 0.185 ± 0.009 | 0.211 ± 0.014 | 0.162 ± 0.014 | 0.150 ± 0.003 |
| **Perfluorodecalin radius (nm)** | 69.23 ± 0.35 | 71.54 ± 0.42 | 72.00 ± 0.35 | 70.11 ± 0.17 |
| **Perfluorodecalin PDI** | 0.140 ± 0.037 | 0.139 ± 0.091 | 0.163 ± 0.017 | 0.165 ± 0.001 |

### Radius and polydispersity index (PDI) of NPs with different 19F compounds

The size stability over time indicates the NPs remain monodisperse and within the desired nanometer scale, a critical parameter for predictable *in vivo* behavior and imaging contrast.

### Example 2.2. Phospholipid composition study

The effect of phospholipid composition on NP size and stability was assessed without DSPE-PEG (**Table 2** and **Figure 4**). Increasing the molar ratio of DSPC and cholesterol enhanced NP stability, optimizing membrane rigidity and thus NP durability.

**Table 2**

| **PFOB Sample** | **DSPC [µmol]** | **Cholesterol [µmol]** |
|---|---|---|
| **5** | 0.95 | 1.0 |
| **6** | 1.40 | 1.50 |
| **7** | 1.90 | 2.00 |
| **8** | 2.40 | 2.50 |
| **9** | 2.85 | 3.00 |
| **10** | 3.35 | 3.50 |
| **11** | 3.80 | 4.00 |
| **12** | 4.275 | 4.50 |
| **13** | 4.75 | 5 |

### Phospholipid composition study

### Example 2.3. Nanoparticle stability at 30 Days

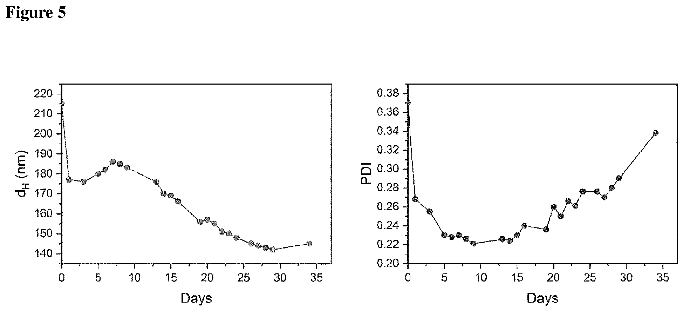

Long-term stability is essential for clinical applications. A formulation with 3.35 mol DSPC, 3.5 mol cholesterol, and 0.175 mol DSPE-PEG was evaluated at 30 days, showing consistent size and low PDI (**Table 3** and **Figure 5**), indicative of robust NP integrity over time.

**Table 3**

| **Sample** | **n_{DSPC} [mol]** | **n_{Cholesterol} [mol]** | **D_{DSPE-PEG} [mol]** | **C_{PFOB} [mol/L]** |
|---|---|---|---|---|
| PFOB | 3.35 | 3.5 | 0.175 | 0.1 |

### Sample evaluated for 30-day stability

### Example 2.4. Summary of NP synthesis with PFOB

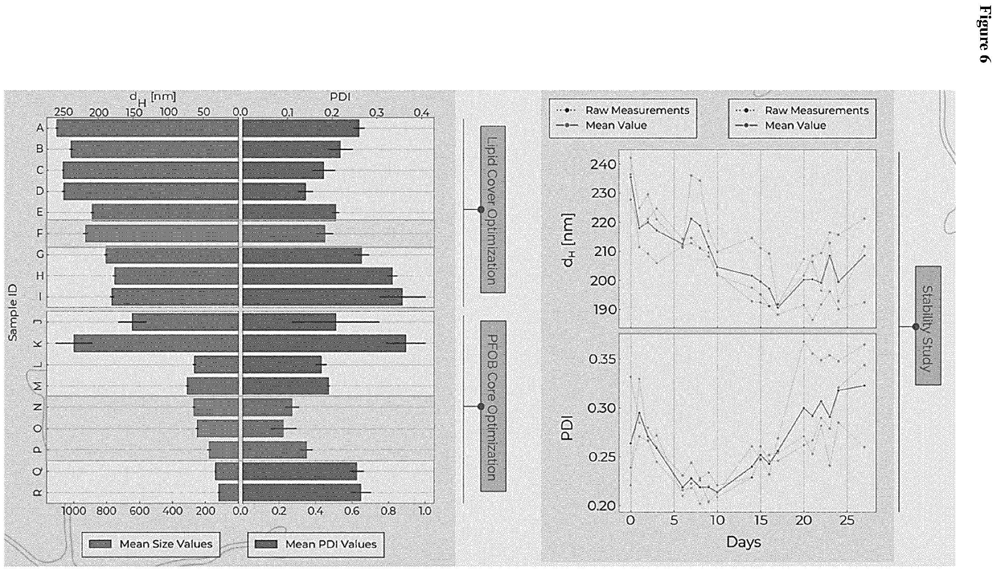

**Table 4** and **Figure 6** summarizes the formulation parameters of PFOB-loaded NPs used for the studies, confirming consistency in synthesis and reproducibility.

**Table 4**

| **C_{PFOB} [mol/L]** | **n_{PFOB} [µmol]** | **n_{DSPC} [mol]** | **n_{Cholesterol} [mol]** | **n_{DSPE-PEG} [mol]** |
|---|---|---|---|---|
| 0.1 - 0.05 | 2.58 - 0.75 | 3.35 | 3.5 | 0.175 |

### Summary of NP synthesis parameters

### Example 2.5. Transmission Electron Microscopy (TEM)

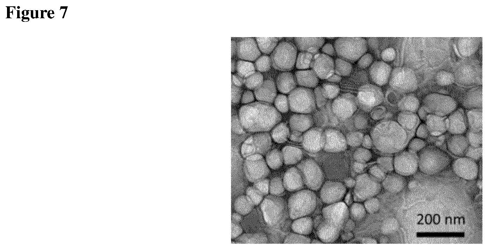

TEM images (**Figure 7**) confirmed spherical morphology of the NPs with radio consistent with DLS data. The spherical shape is beneficial for uniform distribution and controlled pharmacokinetics. Additionally, an estimation of fluorine content suggested approximately 2.05 × 10^7 fluorine atoms per 50 nm radius NP, providing a sufficiently strong 19F MRI signal for sensitive detection.

### Example 2.6. MR Characterization Studies. 19F Quantification and Imaging

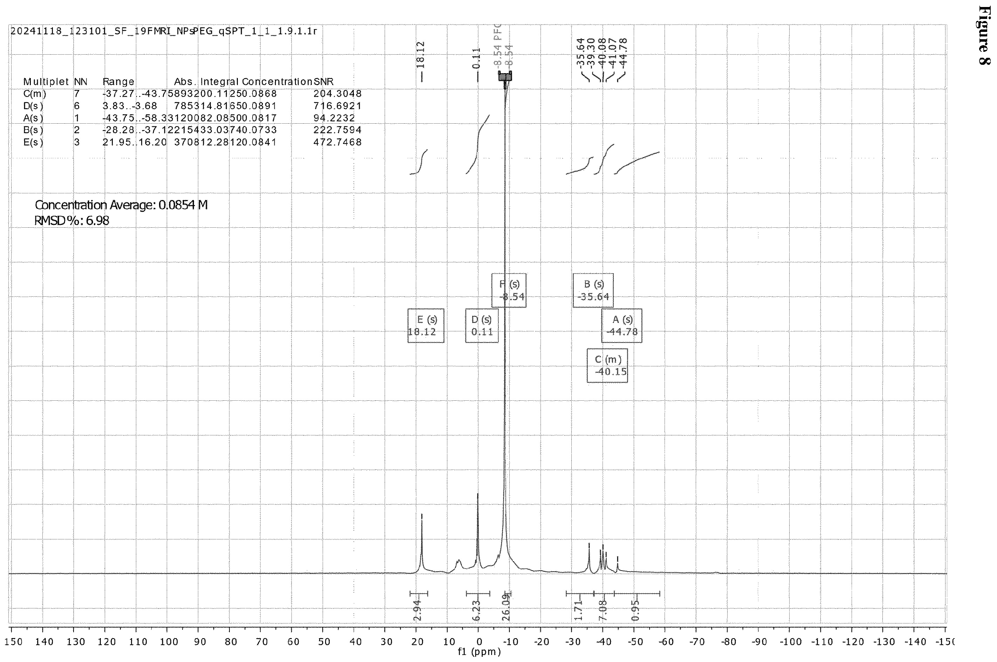

Magnetic resonance spectroscopy was employed to confirm the successful encapsulation of PFOB within the NPs. ¹⁹F signals were clearly detected at concentrations as low as 0.1 mM, confirming the sensitivity of the technique for tracking the fluorinated component (**Figure 8**). This highlights the efficiency of the formulation in preserving and presenting the ¹⁹F nucleus for downstream imaging applications.

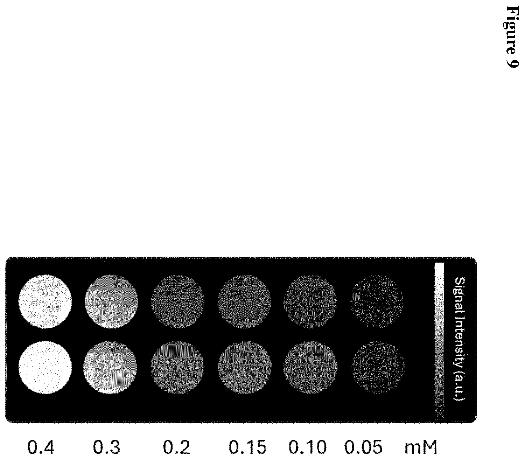

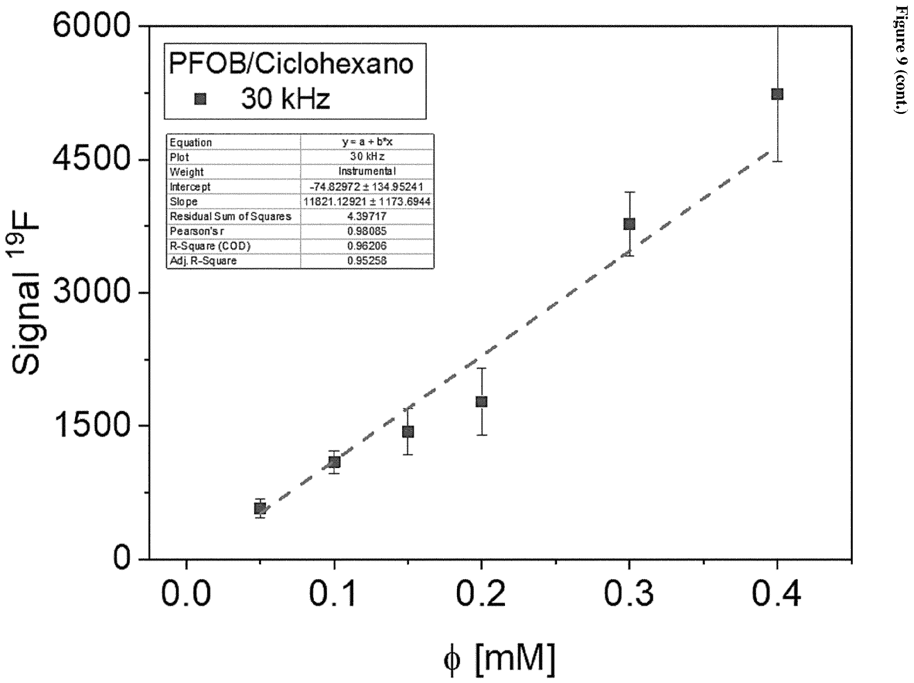

Subsequently, MRI was performed using a 9.4 T Bruker BIOSPEC 94/20 system to evaluate the imaging performance of the fluorinated NPs. The imaging results revealed a robust, linear correlation between the PFOB concentration and the corresponding MR signal intensity across the tested range (**Figure 9**). This linearity underscores the NPs' potential for use as quantitative ¹⁹F MRI contrast agents, where signal intensity can serve as a reliable indicator of local nanoparticle concentration. These findings demonstrate the dual utility of the system for both detection and quantification of targeted regions, supporting its application in high-resolution, quantitative imaging of pathological processes such as thrombus formation or blood-brain barrier disruption.

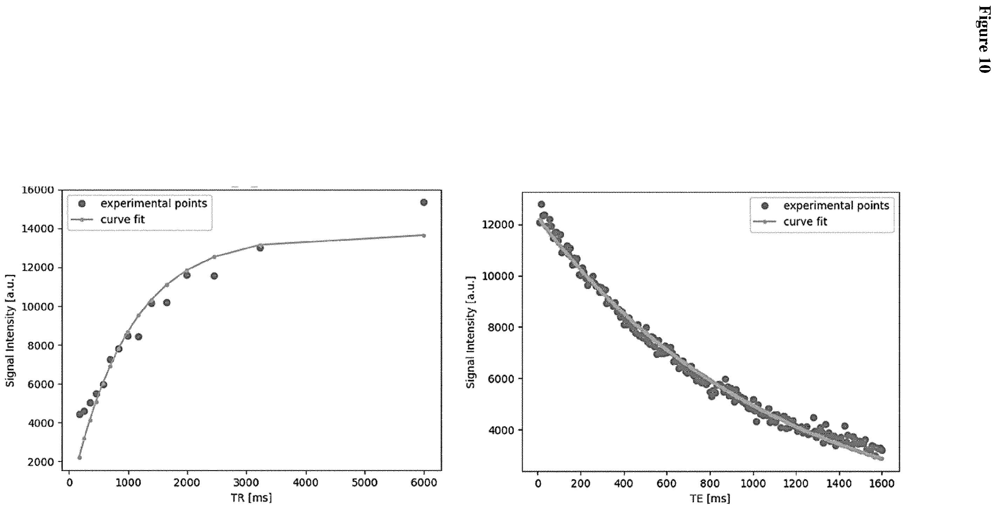

The T1 and T2 relaxation times of the NP suspensions (**Table 5, Table 6** and **Figure 10**) were consistent with efficient contrast generation, critical for high-resolution MRI of thrombi.

**Table 5**

| **Sample** | **n_{DSPC} [mol]** | **n_{Cholesterol} [mol]** | **n_{DSPE-PEG} [mol]** | **C_{PFOB} [mol/L]** |
|---|---|---|---|---|
| PFOB | 3.35 | 3.5 | 0.175 | 0.1 |

### Sample evaluated

**Table 6**

| | **PFOB (Pure)** | **PFOB NPs** |
|---|---|---|
| **T₁ (ms)** | 1623.18 ± 12.24 | 989.79 ± 124.22 |
| **T₂(ms)** | 580.65 ± 2.44 | 1101.46 ± 7.99 |

### T1 and T2 results

### Example 2.7. In vitro stability in whole blood

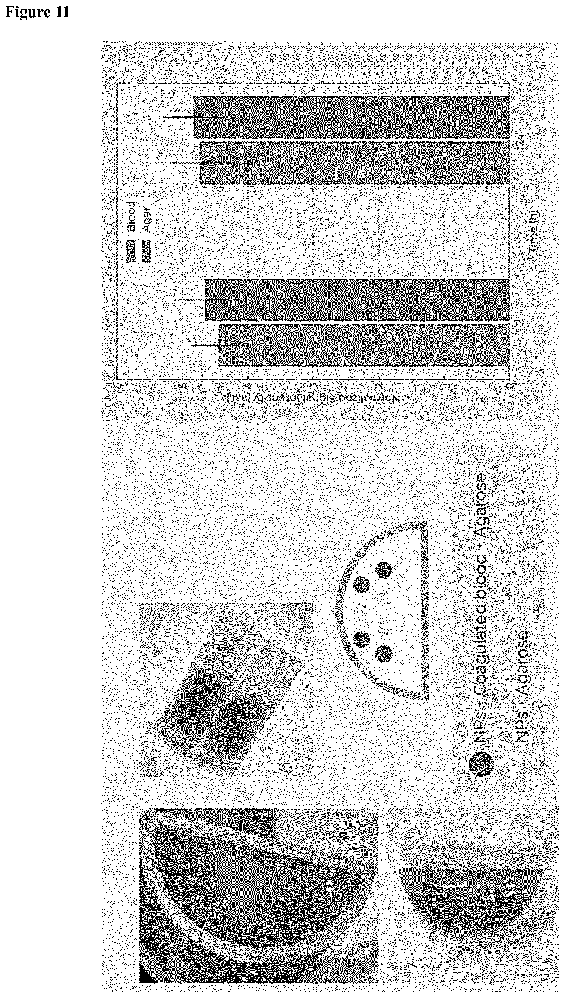

To assess the suitability of the NPs for *in vivo* applications, their ¹⁹F MRI signal stability was evaluated in a biologically relevant environment. Specifically, freshly collected whole blood was used to mimic the complex conditions encountered within the circulatory system. NPs were incubated with blood for 1 hour, after which the resulting ¹⁹F MRI signal was measured using an agarose phantom. This setup enabled direct comparison of signal intensity between blood-incubated samples and control samples. As shown in **Figure 11****,** the signal intensity remained stable, with no significant reduction observed. This finding indicates that key blood components-such as red and white blood cells, platelets, and plasma proteins-do not interfere with the fluorine signal. Importantly, this demonstrates the robustness of the NP formulation and its resistance to signal quenching or aggregation in a physiological matrix. These results support the potential of these ¹⁹F-labeled NPs for reliable and accurate imaging in preclinical and potentially clinical *in vivo* settings.

### Example 2.8. In vitro toxicity and thrombus affinity

Ongoing studies are evaluating the cytotoxicity and thrombus-binding affinity of the nanoparticles using various cell lines, including microvascular endothelial cells (human hCMEC/D3s, rodent bEnd.3), human astrocytes (p10251-1m), and pericytes (cl 05008-clth). These cell models are being used to assess the NPs' potential effects on cellular viability, as well as their ability to bind specifically to thrombus-related antigens, providing insights into the nanoparticles' suitability for targeted imaging and therapeutic applications. Early results indicate low toxicity and promising specific binding to thrombus-related antigens, attributed to the functionalization of NPs with platelet membrane coatings presenting targeting ligands.

### Example 2.9. In vivo imaging efficacy

*In vivo* efficacy studies using thromboembolic stroke models are being initiated to validate the NP formulations as sensitive and specific 19F MRI contrast agents for thrombus detection. The optimized formulations developed here provide a robust platform for non-invasive thrombus imaging, which could enhance stroke diagnosis and treatment monitoring.

## Claims

1. Fluorine-19-containing nanoparticles for use in an *in vivo* method of magnetic resonance imaging (MRI) of thrombi in a subject, wherein the nanoparticles serve as contrast agents.

2. Fluorine-19-containing nanoparticles for use, according to claim 1, in a *in vivo* method for the visualization, localization and/or characterization of thrombi in a subject.

3. Fluorine-19-containing nanoparticles for use, according to any of the previous claims, in a *in vivo* method for the visualization, localization and/or characterization of thrombi during the acute phase of cerebral ischemia in a subject.

4. Fluorine-19-containing nanoparticles for use, according to any of the previous claims, in a *in vivo* method for differentiating between fresh platelet-rich thrombi and older fibrin-rich thrombi in a subject.

5. Fluorine-19-containing nanoparticles for use, according to any of the previous claims, wherein the *in vivo* method comprises: administering the nanoparticles to the subject; acquiring MRI images of the relevant anatomical region; and visualizing, localizing and/or characterizing thrombi based on the acquired images.

6. Fluorine-19-containing nanoparticles for use, according to any of the relevant claims, wherein the nanoparticles are lipid-based nanoparticles.

7. Fluorine-19-containing nanoparticles for use, according to any of the previous claims, wherein the nanoparticles are lipid-based nanoparticles and comprise a matrix encapsulating fluorinated compounds selected from perfluorooctyl bromide (PFOB), perfluorooctane, and perfluorodecalin.

8. Fluorine-19-containing nanoparticles for use, according to any of the previous claims, wherein the lipid matrix comprises 1,2-Distearoyl-sn-glycero-3-phosphocholine (DSPC) and cholesterol.

9. Fluorine-19-containing nanoparticles for use, according to any of the previous claims, wherein the nanoparticles are functionalized with platelet plasma membranes for specific targeting of thrombi.

10. Fluorine-19-containing nanoparticles for use, according to any of the previous claims, wherein the nanoparticles are coated with polyethylene glycol (PEG) to enhance stability, solubility, and biocompatibility.

11. A fluorine-19-containing nanoparticle, comprising:
a. A lipid matrix;
b. A fluorinated compound encapsulated within the lipid matrix;
c. A coating of platelet plasma membranes functionalizing the nanoparticle for targeting thrombi; and
d. Optionally, a polyethylene glycol (PEG) coating to enhance nanoparticle stability and biocompatibility.

12. A fluorine-19-containing nanoparticle according to claim 11, wherein:
a. The lipid matrix comprises 1,2-Distearoyl-sn-glycero-3-phosphocholine (DSPC) and cholesterol;
b. The fluorinated compound encapsulated within the lipid matrix is selected from perfluorooctyl bromide (PFOB), perfluorooctane, and/or perfluorodecalin;
c. The nanoparticle is functionalized with platelet plasma membranes for targeting thrombi; and
d. Optionally, the nanoparticle is coated with polyethylene glycol (PEG) to enhance stability and biocompatibility.

13. A method of preparing fluorine-19-containing nanoparticles, comprising:
a. Preparing a lipid mixture;
b. Encapsulating a fluorinated compound within the lipid mixture;
c. Functionalizing the formed nanoparticles with platelet plasma membranes; and
d. Optionally coating the nanoparticles with polyethylene glycol (PEG).

14. A method of preparing fluorine-19-containing nanoparticles according to claim 13, wherein:
a. The lipid mixture comprises 1,2-Distearoyl-sn-glycero-3-phosphocholine (DSPC) and cholesterol;
b. The fluorinated compound encapsulated within the lipid mixture is selected from perfluorooctyl bromide (PFOB), perfluorooctane, or perfluorodecalin, preferably by nanoprecipitation;
c. The formed nanoparticles are functionalized with platelet plasma membranes; and
d. Optionally, the nanoparticles are coated with polyethylene glycol (PEG).
